# FASCICULE DE BREVET EUROPEEN

(11) **EP 1 432 414 B1**
(45) Date de publication et mention de la délivrance du brevet: **23.08.2006**
(21) Numéro de dépôt: 02800625.2
(22) Date de dépôt: 03.10.2002
(51) Int. Cl.: A61K 31/4436, A61K 47/10, A61K 47/14, A61K 47/30, A61P 17/06

(54) **VERNIS A ONGLES CONTENANT DU TAZAROTENE ET SON UTILISATION DANS LE TRAITEMENT ET/OU LA PREVENTION DU PSORIASIS**
NAGELLACK ENTHALTEND TAZAROTEN UND VERWENDUNG DESSEN ZUR BEHANDLUNG UND/ODER ZUR PRÄVENTION VON PSORIASIS
NAIL VARNISH CONTAINING TAZAROTENE AND USE THEREOF FOR TREATING AND/OR PREVENTING PSORIASIS

(30) Priorité: 05.10.2001 FR 0112825
(43) Date de publication de la demande: 30.06.2004
(60) Demande divisionnaire: 06114290.7
(73) Titulaire: Pierre Fabre Dermo-Cosmétique, 92100 Boulogne-Billancourt (FR)
(72) Inventeur: LEFRANCOIS, Pascal, 81500 Lavaur (FR); NAVARRO, Roger Riveneuve de Crieu, F-09100 Pamiers (FR); DELAUNOIS, Marlène, F-31290 Villefranche-du-Lauragais (FR)
(74) Mandataire: Ahner, Francis
(86) Numéro de dépôt international: PCT/FR2002/003366
(87) Numéro de publication internationale: WO 2003/030896

(56) Documents cités:
- WO-A-01/01932
- WO-A-98/58671
- US-A- 4 250 164
- US-A1- 2001 006 625
- "Treating Fingernail Psoriasis With Tazarotene 0.1% Gel" INTERNET, [en ligne] 19 juin 2001 (2001-06-19), XP002194590 Extrait de l'Internet: <URL:http://www.sdefmail.com/Psoriasis/IPS /2001d6.htm> [extrait le 2002-03-27]

## Description

La présente invention a pour objet l'utilisation du tazarotène pour la préparation d'un vernis à ongles pour le traitement et/ou la prévention du psoriasis, un vernis à ongles contenant du tazarotène et un procédé de fabrication.

Le psoriasis est une maladie de la peau qui touche plus de deux pour cent des populations occidentales.

Elle se traduit par une dermatose érythématosquameuse chronique. La lésion associe des anomalies du kératinocyte (activité mitotique fortement augmentée et différenciation anormale) à des phénomènes inflammatoires du derme et de l'épiderme.
La maladie est sous la dépendance de facteurs génétiques révélés par divers facteurs de l'environnement.

Les affections des ongles dues au psoriasis sont des formes de maladies tenaces qu'il n'a pas été possible jusqu'à présent de traiter de façon satisfaisante. Elles touchent, selon les pays entre 10 et 78% des populations développant un psoriasis.

Aujourd'hui, le traitement le plus répandu du psoriasis de l'ongle consiste en des injections sous-cutanées de corticoïdes. Ces corticoïdes sont des principes actifs anti-inflammatoires largement utilisés pour les traitements dermatologiques.

Ces injections très douloureuses peuvent être accompagnées d'un traitement topique.

Un autre traitement consiste à appliquer sur les ongles localement des substances spécifiques à action antipsoriasique sous forme de crème. Dans ce domaine, on a essayé les méthodes de traitement les plus diverses. Ainsi, dans un traitement combiné on a d'abord traité les ongles avec des solutions de substances à action antipsoriasique et on a appliqué des pansements avec de la crème pendant la nuit. Cette méthode de traitement est également fort désagréable et psychologiquement éprouvante pour les patients. D'une part, le traitement des ongles avec des solutions est nécessaire plusieurs fois par jour. D'autre part, ceux-ci doivent être munis de pansements, surtout la nuit. En conséquence, le traitement, qui dure habituellement plusieurs mois, n'est pas fréquemment poursuivi par les patients qui au contraire se découragent et deviennent négligents. Cela conduit à l'échec du traitement. Dans cette méthode, le succès du traitement est en outre compromis par le fait que les solutions et les crèmes sont habituellement miscibles à l'eau ou hydrophiles, et peuvent être éliminées de la surface de l'ongle ou entraînées par dissolution hors de l'ongle lors de la toilette, de bains et de douches, et doivent donc être ensuite appliquées à nouveau.

Le tazarotène est un dérivé rétinoïde connu pour son activité antipsoriasique.

Il s'agit d'un dérivé de rétinoide également nommé 6-[(3,4-Dihydro-4,4-diméthyl-2H-1-benthiopyran-6-yl)éthynyl]-3-pyridinecarboxylate d'éthyle de formule

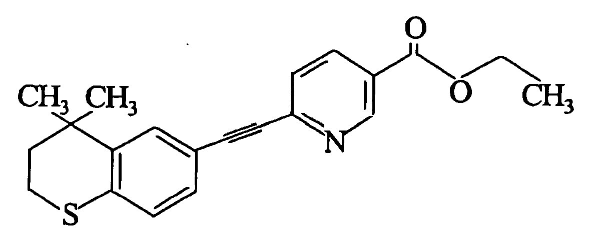

Il peut notamment se présenter sous forme de crème ou de gel.
Cependant si l'efficacité du tazarotène sur des plaques de psoriasis a été bien démontrée, traiter un psoriasis qui se développe sous l'ongle est délicat.
Il est difficile d'atteindre des concentrations efficaces de principe actif pour éliminer le psoriasis dans ces conditions.

La demande de brevet US-2001/0006625 décrit un vernis à ongle contenant des glucocorticoïdes et un agent filmogène. Le brevet US-4,250,164 divulgue des vernis à ongle utilisés pour le traitement des psoriasis obtenus en ajoutant un corticostéroïde à une composition de vernis connue.

On a maintenant trouvé qu'il était possible de traiter des psoriasis des ongles avec succès et/ou de les prévenir en appliquant sur les ongles, et notamment sur les ongles atteints de psoriasis, le vernis à ongles selon l'invention.

La présente invention a donc pour objet un vernis à ongles à action antipsoriasique contenant- :
- du tazarotène,
- un agent filmogène soluble ou insoluble dans les solvants polaires.
- un solvant physiologiquement acceptables constitué d'un mélange d'acétate d'éthyle et d'alcool isopropylique.

La présente invention a aussi pour objet un vernis à ongles tel que défini ci-dessus contenant en outre un ou des agents de pénétration ainsi qu'éventuellement, des additifs utilisés habituellement dans des cosmétiques.

Un autre objet de l'invention est l'utilisation de tazarotène pour la préparation d'un vernis à ongles tel que défini ci-dessus pour le traitement et/ou la prévention du psoriasis.

Selon l'invention le vernis à ongles doit contenir suffisamment de principe actif afin que son dépôt sur l'ongle permette une libération convenable assurant une diffusion jusqu'au lit de l'ongle et permettant ainsi d'atteindre le seuil thérapeutique.

Un autre objet de la présente invention est un procédé de fabrication du vernis à ongles selon l'invention qui comprend une étape de mélange d'un agent filmogène et de tazarotène. Avantageusement, on effectue la dissolution du principe actif et des autres constituants éventuellement présents dans le ou les solvants avant l'agent filmogène.
Les vernis à ongles selon l'invention peuvent contenir des agents filmogènes solubles dans des solvants hydroalcooliques ou des solvants non polaires qui après séchage sur l'ongle, forment des films résistants à l'eau.

Conviennent en tant qu'agents filmogènes solubles dans les solvants non polaires (ou insoluble dans les solvants polaires), des substances à base de nitrate de cellulose ou de polymères synthétiques bien connus du formulateur pour leur innocuité. On peut citer par exemple, le polyacrylate de vinyle et le polyacrylate de vinyle partiellement saponifié, des copolymères d'acétate de vinyle d'une part et d'autre part, d'acide acrylique ou d'acide crotonique ou d'un maléate de monoalkyle, des copolymères ternaire d'acétate de vinyle d'une part et d'autre part, d'acide crotonique et de néodécanoate de vinyle ou d'acide crotonique et de propionate de vinyle, des copolymères d'éther méthyl vinylique et d'un maléate de monoalkyle, en particulier sous forme de maléate de monobutyle, des copolymères d'esters vinyliques d'acides gras et d'acide acrylique ou d'acide méthacryliques, des copolymères de N-vinylpyrrolidone, acide méthacrylique et méthacrylate d'alkyle, des copolymères d'acide acrylique et d'acide méthacrylique, ou d'acrylate d'alkyle ou de méthacrylate d'alkyle, des polyacétals de vinyle et des poly butyrals de vinyle, des poly-N-vinylpirrolydones substitués par des groupes alkyles des esters alkyliques de copolymères d'oléfine et d'anhydride maleique et des produits de réaction de colophane et d'acide acrylique. Dans les esters, les restes d'alkyle sont habituellement des chaînes courtes et ne contiennent en général pas plus de quatre atomes de carbone.

Conviennent en tant qu'agents filmogènes solubles dans des solvants polaires les polyacrylamides; les copolymère acrylique/méthacrylique, polymethacrylate/butylacrylate, acrylique/acrylate; l'alcool polyvinylique; le copolymère polyvinylméthyl ether/anhy-dride maléique; la polyvinylpyrrolidone; le copolymère polyvinylpyrrolidone/vinyl acétate et le copolymère vinylpyrrolidone/diméthylaminethyl méthacrylate.

Un mélange d'agents filmogènes de la même classe (soluble dans les solvants polaires ou bien soluble dans les solvants non polaires) peut être utilisé à titre d'agent filmogène dans le cadre de l'invention.

On préfère parmi les agents filmogènes solubles dans les solvants polaires les copolymères acryliques/acrylates et acrylique/méthacrylates ou le ter copolymère acrylique/acrylate/méthacrylate.

On préfère parmi les agents filmogènes solubles dans les solvants non polaires, le copolymère éther méthyl vinylique/maléate de monobutyle.

Lorsqu'on utilise à titre d'agent filmogène un agent filmogène soluble dans l'eau, on peut utiliser comme solvant, dans le cadre de la présente invention, des alcools tels que l'éthanol, l'alcool isopropylique ou tout autre alcool ayant un point d'ébullition suffisamment bas pour avoir un temps de séchage très court. Ces alcools peuvent être présents dans le vernis sous forme de mélanges et une quantité d'eau allant de 0 à 20% en poids par rapport à la quantité totale de solvant peut être alors ajoutée. Il s'agit des solvants dits solvants hydroalcooliques.

Dans le cadre de la présente invention et que l'agent filmogène soit soluble ou insoluble dans l'eau, on préfère utiliser une association de solvants ou système de solvants. Ces solvants ont une importance prépondérante pour le temps de séchage, la facilité d'application au pinceau et d'autres propriétés importantes du vernis ou de la pellicule de vernis. Le système de solvants peut être compris entre 70 et 94% en poids par rapport au poids total du vernis.

Ce système de solvant est de préférence un mélange de solvants à bas point d'ébullition (= point d'ébullition jusqu'à 100 °C) et de solvants à point d'ébullition moyen (= point d'ébullition jusqu'à 150°C), éventuellement avec une faible proportion de solvants à point d'ébullition élevé (= point d ébullition jusqu'à 200°C).

A titre d'agents de pénétration on peut notamment citer l'urée, l'acide oléique et l'éthoxy diglycol. Tout autre agent de pénétration utilisé couramment dans ce type de formulations pourra l'être également dans le cadre de la présente invention.

Les vernis à ongles selon l'invention peuvent contenir en outre des additifs utilisés couramment dans des cosmétiques, tels que plastifiants à base de phtalate, d'urée ou de camphre, de colorants ou de pigments colorés, agent nacrant, retardateurs de sédimentation, résines sulfonamide, silicates, parfums, agents mouillants, tels que le dioctylsulfosuccinate de sodium, dérivés de la lanoline, agents de protection contre la lumière, tels que la 2-hydroxy-4-méthoxybenzophénone, substances à action antibactérienne et substances à action kératolytique et/ou kératoplastique, telles que le sulfite d'ammonium, des esters et sels de l'acide thioqlycolique, l'urée, l'allantoine, des enzymes et l'acide salicylique.

Les vernis à ongles colorés ou pigmentés offrent par exemple l'avantage que la composition selon l'invention peut être adaptée au sens esthétique du patient.

La préparation du vernis à ongles est effectuée de la façon usuelle par mélange des composants individuels et, si nécessaire un traitement ultérieur adapté à chaque composition.

Dans le vernis à ongles selon l'invention, le tazarotène est en général contenu en une quantité allant de 0,1 à 4%, de préférence de 0,1% à 2% en poids par rapport au poids total de la composition.

Le vernis à ongles selon la présente invention contient en général de 0,1 à 30%, de préférence de 5 à 20% en poids d'agent filmogène, de 69 à 99% en poids, de préférence de 79 à 94% et plus précisément de 81 à 86% en poids de solvant et de 0,5 à 15% en poids, de préférence de 1 à 10% et plus précisément de 3 à 7% d'agent de pénétration par rapport au poids total du vernis.

Les proportions citées ci-dessus permettent notamment d'obtenir un vernis adapté pour la chaîne de production tout en présentant des propriétés de facilité à l'étalement et un séchage rapide.

Il est connu que les couches cornées superficielles ont, entre autres, la fonction biologique d'empêcher la pénétration de substances étrangères. Les compositions selon l'invention permettent le passage en proportion considérable du principe actif à travers les 168 couches cornées superficielles et exercent ainsi une action durable en profondeur.

La présente invention concerne par ailleurs, à titre de mode particulier de réalisation, un vernis tel que décrit précédemment contenant en outre un corticoïde ou un mélange de différents corticoïdes.

Les corticoïdes pouvant être associés au tazarotène peuvent être choisis parmi les corticoïdes de :
- très forte activité, parmi lesquels le propionate de clobétasol ou le dipropionate de bétaméthasone ;
- forte activité, parmi lesquels : le valérate ou dipropionate de bétaméthasone, l'acétonide de fluocinolone et l'acéponate ou butyrate d'hydrocortisone ;
- assez forte activité, parmi lesquels : l'acétonide de fluocinolone, le désonide ;
- activité modérée, parmi lesquels : l'acétate d'hydrocortisone.

On préfère, à titre de vernis à ongles contenant un corticoïde, un vernis à ongles contenant le clobétasol ou un de ses sels pharmaceutiquement acceptable, tel que le propionate de clobétasol.

Cette classification des corticoïdes est européenne et non limitative pour l'invention. La quantité de corticoïde peut varier de 0,01% à 5% en poids par rapport au poids total du vernis en fonction de la gravité du psoriasis à traiter.

La présente invention est illustrée plus en détail à l'aide de l'exemple qui suit.

Exemple : Etude de l'activité de formulations de vernis selon l'invention.

Dans les essais sur le pouvoir de pénétration, on a testé plusieurs formules préparées avec des agents filmogènes à tendance hydrophobe dissous dans des solvants très peu polaires.

Nous nous sommes préoccupés de la non solubilisation après séchage dans l'eau du film formé ce qui entraînerait au cours de la toilette la disparition du principe actif et donc la perte de l'activité du produit.

Une première série d'essais a été menée sur des explants de peau afin de vérifier sur ce modèle classique, si nous arrivions à discriminer plusieurs formules différentes.
Sur de la peau abdominale humaine dermatomée à 400 microns, en utilisant des cellules de diffusion de type cellules de Franz modifiées, nous avons appliqué les différentes formules en doses finies soit 9,2 mg/cm². L'étude de passage s'est effectuée sur 24h. Le principe actif tazarotène a diffusé à travers la peau et a été collecté dans le liquide récepteur à 3h, 6h, 12h et 24h.

Au bout de 24h, l'actif a été dosé dans le tissu, à la fin de la phase de diffusion (derme et épiderme) et à la surface du tissu, après avoir nettoyé en surface le vernis.

Trois types de formules ont été testés :

| | Formulaires apolaires (en %) | | | |
|---|---|---|---|---|
| | 4 | 5 | 6 | Temoin gel |
| TAZAROTENE | 1 | 0,5 | 0,1 | 0,1 |
| B.H.T | 0,05 | 0,05 | 0,05 | - |
| COPOLYMERE: ETHER METHYL VINYLIQUE MONOBUTYL ESTER D'ACIDE MALEIQUE | 15 | 15 | 15 | - |
| | | | | |
| ISOPRANOL | 50,15 | 50,45 | 50,69 | - |
| ACETATE D'ETHYLE | 33,80 | 34,00 | 34,16 | - |

| | | | | |
|---|---|---|---|---|
| B.H.T : Butyl hydroxy toluene | | | | |

Nous avons utilisé un témoin du commerce, connu sous la marque ZORAC® , gel dermique dosé à 0,1% de tazarotène.

**Etude du passage transcutané sur 24 heures du tazarotène.**

| | Caractéristique | Passage en micro grammes |
|---|---|---|
| Formule 4 | Vernis lipophile tazarotène 1% | 0,12 |
| Formule 5 | Vernis lipophile tazarotène 0,5% | 0,065 |
| Formule témoin | Gel tazarotène 0,1% | 0,012 |

| | | |
|---|---|---|
| Résultats : Nous avons démontré que plus la concentration en actif est importante, plus le principe actif pénètre. | | |

**Une deuxième série d'essais** de pénétration a été conduite sur la corne de boeuf.
Ces cornes de boeufs ont été découpées de façon à obtenir des disques de kératine de diamètre compatible avec celui des cellules de Franz, ces disques ayant une épaisseur standardisée à 0,6 mm.

La cellule de Franz utilisée dans cette expérimentation se présente comme un récipient à double compartiment séparé par la peau ou la corne. Dans le compartiment inférieur, se trouve une solution de sérum physiologique thermostatée à 37°C. Le produit à étudier est déposé dans le récipient supérieur, au contact de la peau ou de la corne. Des prélèvements réguliers sont effectués dans le compartiment inférieur, pour étudier les quantités de principe actif.

Le vernis contenant le tazarotène est déposé sur ce disque de corne et le passage trans ongle est suivi en utilisant du tazarotène marqué au C14. La méthode d'étude du pouvoir de pénétration sur de la corne de boeuf permet d'étudier les composés en ce qui concerne leur pouvoir de pénétration à une concentration efficace vis-à-vis de la corne dont la composition est très semblable à la composition de l'ongle.

Sur la corne de boeuf sont déposés 7,1 mg/cm² de vernis tels que définis dans les formules 4 et 5.

Le temps de contact a été de 48 heures avec des prélèvements à 6h, 12h, 24h, 36h et 48h. L'ongle à été dermatomé en lames de 40 microns, la surface de la corne et la base de la corne, faisant 240 microns.

**Etude du passage trans ongle de 24 heures du tazarotène**

| | caractéristique | Passage en micro grammes |
|---|---|---|
| Formule 4 | Vernis lipophile tazarotène 1% | 2 |
| Formule 5 | Vernis lipophile tazarotène 0,5% | 0,025 |

**En conclusion** : Les vernis à ongles contenant un agent filmogène et un solvant selon l'invention, à base de solvants non polaires, ont un passage à travers l'ongle qui permet d'atteindre le seuil thérapeutique.

Ces vernis permettent d'avoir une efficacité rapide et indolore contrairement aux autres thérapies connues dans cette affection.

## Revendications

1. Vernis à ongles à action antipsoriasique **caractérisé en ce qu'**il contient :
- du tazarotène,
- un agent filmogène soluble ou insoluble dans des solvants polaires,
- un solvant physiologiquement acceptable constitué d'un mélange d'acétate d'éthyle et d'alcool isopropylique.

2. Vernis à ongles à action antipsoriatique selon la revendication 1, **caractérisé en ce qu'**il contient un ou des agents de pénétration, ainsi qu'éventuellement des additifs utilisés habituellement dans des cosmétiques.

3. Vernis à ongles selon la revendication 2,
**caractérisé en ce que** l'agent de pénétration est choisi parmi l'urée, l'acide oléique et l'étoxy diglycol.

4. Vernis à ongles selon l'une quelconque des revendications 1 à 3, **caractérisé en ce qu'**il contient le tazarotène en une quantité allant de 0,1 à 4% en poids par rapport au poids total de la composition.

5. Vernis à ongles selon l'une quelconque des revendications 1 à 4, **caractérisé en ce que** le solvant est présent en une quantité comprise entre 69 et 99% en poids.

6. Vernis à ongles selon l'une quelconque des revendications 1 à 5, **caractérisé en ce que** l'agent filmogène est choisi parmi les copolymères acryliques/acrylates, acryliques/méthacrylate, acryliques/acrylates/méthacrylates, éther méthylvinyliques/maléate de monobutyle.

7. Procédé pour la préparation d'un vernis à ongles selon l'une quelconque des revendications 1 à 6, **caractérisé en ce que** qu'il contient une étape de mélange d'un agent filmogène soluble ou insoluble dans l'eau avec le tazarotène ainsi qu'éventuellement avec d'autres composants usuels pour la préparation de vernis à ongles.

8. Vernis à ongles selon l'une quelconque des revendications 1 à 6, **caractérisé en ce qu'**il contient en outre un corticoïde ou un mélange de différents corticoïdes.

9. Vernis à ongles selon la revendication 8**, caractérisé en ce que** le corticoïde peut être choisi parmi le propionate de clobétasol ou le dipropionate de bétaméthasone, le valérate ou dipropionate de bétaméthasone, l'acétonide de fluocinolone et l'acéponate ou butyrate d'hydrocortisone, l'acétonide de fluocinolone, le désonide, l'acétate d'hydrocortisone.

10. Vernis à ongles selon la revendication 9, **caractérisé en ce que** le corticoïde est le clobétasol ou un de ses sels pharmaceutiquement acceptable.

11. Utilisation du tazarotène pour la préparation d'un vernis à ongles selon l'une des revendications 1 à 6, destiné au traitement du psoriasis.

## Claims

1. Nail varnish with antipsoriatic action, **characterized in that** it contains:
- tazarotene,
- a film-forming agent which is soluble or insoluble in polar solvents,
- a physiologically acceptable solvent consisting of a mixture of ethyl acetate and isopropylic alcohol.

2. Nail varnish with antipsoriatic action according to Claim 1, **characterized in that** it contains one or more penetration agents and, optionally, additives conventionally used in cosmetics.

3. Nail varnish according to Claim 2, **characterized in that** the penetration agent is chosen from urea, oleic acid and ethoxydiglycol.

4. Nail varnish according to any one of Claims 1 to 3, **characterized in that** it contains tazarotene in an amount ranging from 0.1 to 4% by weight relative to the total weight of the composition.

5. Nail varnish according to any one of Claims 1 to 4, **characterized in that** the solvent is present in an amount of between 69% and 99% by weight.

6. Nail varnish according to any one of Claims 1 to 5, **characterized in that** the film-forming agent is chosen from acrylic/acrylate, acrylic/methacrylate, acrylic/acrylate/methacrylate and methyl vinyl ether/monobutyl maleate copolymers.

7. Method for preparing a nail varnish according to any one of Claims 1 to 6, **characterized in that** it contains a step consisting in mixing a water-soluble or
- insoluble film-forming agent with tazarotene and, optionally, with other usual components for preparing nail varnishes.

8. Nail varnish according to any one of Claims 1 to 6, **characterized in that** it also contains a corticoid or a mixture of various corticoids.

9. Nail varnish according to Claim 8, **characterized in that** the corticoid can be chosen from clobetasol propionate or betamethasone dipropionate, betamethasone valerate or dipropionate, flucinolone acetonide and hydrocortisone aceponate or butyrate, flucinolone acetonide, desonide, and hydrocortisone acetate.

10. Nail varnish according to Claim 9, **characterized in that** the corticoid is clobetasol or one of its pharmaceutically acceptable salts.

11. Use of tazarotene for preparing a nail varnish according to one of Claims 1 to 6, intended for treating psoriasis.

## Patentansprüche

1. Nagellack mit Antipsoriasis-Wirkung, **dadurch gekennzeichnet, dass** er enthält:
- Tazaroten,
- ein in polaren Lösungsmitteln lösliches oder unlösliches filmbildendes Mittel,
- ein physiologisch annehmbares Lösungsmittel, das aus einer Mischung von Ethylacetat und Isopropylalkohol besteht.

2. Nagellack mit Antipsoriasis-Wirkung nach Anspruch 1, **dadurch gekennzeichnet, dass** er ein oder mehrere Penetrationsmittel sowie gegebenenfalls Zusätze enthält, die üblicherweise in der Kosmetik verwendet werden.

3. Nagellack nach Anspruch 2, **dadurch gekennzeichnet, dass** das Penetrationsmittel aus Harnstoff, Ölsäure und Ethoxydiglycol ausgewählt ist.

4. Nagellack nach irgendeinem der Ansprüche 1 bis 3, **dadurch gekennzeichnet, dass** er Tazaroten in einer Menge von 0,1 bis 4 Gew.-%, bezogen auf das Gesamtgewicht der Zusammensetzung, enthält.

5. Nagellack nach irgendeinem der Ansprüche 1 bis 4, **dadurch gekennzeichnet, dass** das Lösungsmittel in einer Menge zwischen 69 und 99 Gew.-% einschließlich vorliegt.

6. Nagellack nach irgendeinem der Ansprüche 1 bis 5, **dadurch gekennzeichnet, dass** das filmbildende Mittel aus Acryl/Acrylat-, Acryl/Methacrylat-, Acryl/Acrylat/Methacrylat-, Methylvinylether/Monobutylmaleat-Copolymeren ausgewählt ist.

7. Verfahren zur Herstellung eines Nagellacks nach irgendeinem der Ansprüche 1 bis 6, **dadurch gekennzeichnet, dass** es einen Schritt des Mischens eines in Wasser löslichen oder unlöslichen filmbildenden Mittels mit Tazaroten sowie gegebenenfalls mit anderen für die Herstellung von Nagellack üblichen Bestandteilen enthält.

8. Nagellack nach irgendeinem der Ansprüche 1 bis 6, **dadurch gekennzeichnet, dass** er darüber hinaus ein Corticoid oder eine Mischung verschiedener Corticoide enthält.

9. Nagellack nach Anspruch 8, **dadurch gekennzeichnet, dass** das Corticoid aus Clobetasolpropionat oder Betamethasondipropionat, Betamethasonvalerat oder -dipropionat, Fluocinolonacetonid und Hydrocortisonaceponat oder -butyrat, Fluocinolonacetonid, Desonid, Hydrocortisonacetat ausgewählt sein kann.

10. Nagellack nach Anspruch 9, **dadurch gekennzeichnet, dass** das Corticoid Clobetasol oder eines seiner pharmazeutisch annehmbaren Salze ist.

11. Verwendung von Tazaroten für die Herstellung eines Nagellacks nach irgendeinem der Ansprüche 1 bis 6, der zur Behandlung von Psoriasis bestimmt ist.
